# EUROPEAN PATENT APPLICATION

(11) **EP 1 755 058 A2**
(43) Date of publication of application: **21.02.2007**
(21) Application number: 06254164.4
(22) Date of filing: 08.08.2006
(51) Int. Cl.: G06F 19/00

(54) **System and method for the graphical display of patient data**

(30) Priority: 10.08.2005 US 200683
(71) Applicant: Honeywell International, Inc., Morristown, New Jersey 07962-1057 (US)
(72) Inventor: Plocher, Thomas A., Hugo Minnesota 55038 (US)
(74) Representative: Vigars, Christopher Ian

(57) **Abstract**

A system accumulates trend related physiological information pertaining to an individual. Non-textual icons can be used to graphically present trend and condition information to the individual.

## Description

### FIELD OF THE INVENTION

The invention pertains to systems and methods for presentation of information pertaining to trends. More particularly, the invention pertains to such systems and methods where trend information is presented using easy to understand graphics.

### BACKGROUND OF THE INVENTION

Users of known telehealth systems measure one or more their own vital signs at home on a daily basis and, in some case such as diabetics, several times each day. These measurements can include blood pressure, heart rate, oxygen saturation, peak flow body weight, and blood glucose. In addition, by means of activity sensors placed in the home or on the body, telehealth systems are capable of recording client activity level and amount of restful sleep. An ability of these systems to remotely monitor access to medication containers provides an opportunity to record the client's compliance with a prescribed medication schedule.

Daily feedback to residents about these measured health parameters could prove to be an extremely important tool in home-based self-management programs for chronic illnesses. However, presenting numeric readings for these health parameters to elderly people can be fraught with problems. Numeric readings are easily misinterpreted without a sound knowledge of limits within which they should fall. The latter will vary depending on the individual client and the stage of progression of his/her chronic illness. Frail elderly may have neither the expertise nor the cognitive energy to readily interpret five or six health parameter numbers on a daily basis.

### BRIEF DESCRIPTION OF THE DRAWINGS

Fig. 1 is a block diagram of a system in accordance with the invention;

Fig. 2 is a display of physiological parameter identifying icons as well as respective condition indicating icons in accordance with the invention;

Fig. 3 is a display of the various icons of Fig. 2 in accordance with the invention;

Fig. 4 illustrates interaction by a resident with the display of Fig. 3; and

Fig. 5 illustrates explanatory information presented to the resident in response to a parameter selection as in Fig. 4.

### DETAILED DESCRIPTION OF THE INVENTION

While this invention is susceptible of embodiment in many different forms, there are shown in the drawing and will be described herein in detail specific embodiments thereof with the understanding that the present disclosure is to be considered as an exemplification of the principles of the invention and is not intended to limit the invention to the specific embodiments illustrated.

A system in accordance with the invention provides a highly intuitive graphical daily display of health parameters. Displays that embody the invention are completely graphical rather than numeric. Significant deviations from the "normal" range for any given health parameter can be highlighted.

It is an advantage of embodiments of the invention that the resident needs to have no knowledge of "normal" or "abnormal". He/she does not have to interpret measurement numbers. The resident only needs to note from a quick glimpse at the iconic micrographs that one or more parameters is out of range. This tells him/her what needs to improve. This might prompt a question or phone call to a caregiver to ask why.

It is a further advantage that no text labels need be used. Each measured health parameter can be labeled with an intuitive self-explanatory icon rather than a text label.

Text names like "oxygen saturation" are meaningless to most elderly clients. They can be replaced by an icon that depicts the health function reflected in the parameter (in this case the number of red blood cells colored red rather than blue).

In yet another aspect of the invention, the iconic micrographs can be scaled down to require little space on a screen. A tremendous amount of highly relevant health information can be presented in a very small space.

Health parameters that are in a normal range can be shown as green bars. Each parameter that is out of normal range can be shown by a horizontal bar elongated beyond the green "normal" range. Out of normal parameters can be colored, for example, yellow, to distinguish them from normal. The shade of yellow can be graded from light to dark to indicate extent of deviation.

The color can be reinforced by making the length of the yellow bar proportional to the degree of deviation. Waking up in the morning and seeing all green bars tells the resident in an instant that he/she is following the program and everything is normal. Seeing at a glance that one or more parameters is out of a normal range tells them what needs to improve.

The description or name of each health parameter can be displayed as an icon rather than as a textual label. The icons are designed to depict the common health function reflected by the parameter not how it is measured. For example the textual name "oxygen saturation" might be replaced by an icon that shows red blood cells passing through a tube.

The bars in the graphic can be interactive. On a touch screen, for example, each horizontal bar could be touch-interactive. Touching the bar would pop up an explanation of the parameter in plain language, why the deviation might have occurred, and what to do about it.

It will be understood by those of skill in the art that the above described invention is not limited to rectangular icons. Other shapes come within the spirit and scope of the invention.

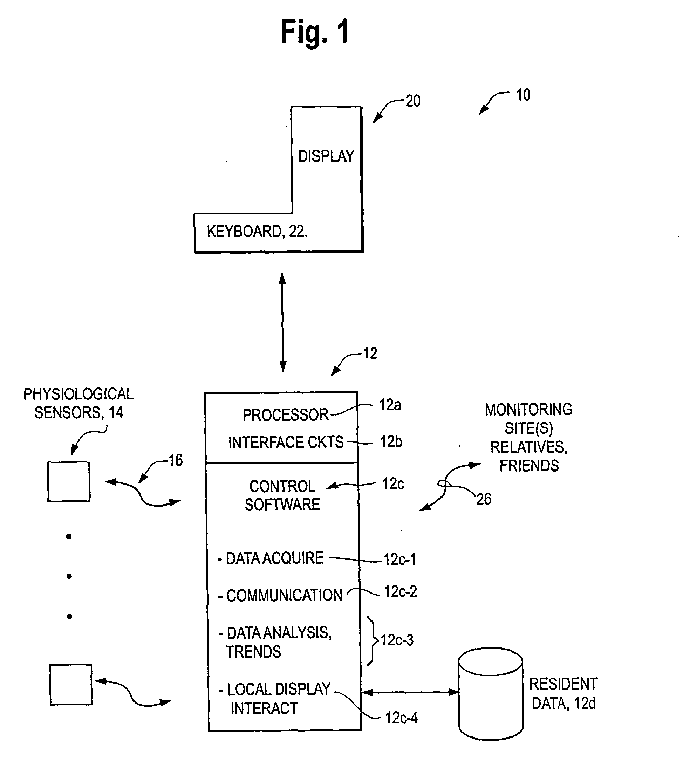

Fig 1. illustrates various aspects of a system 10 which embodies the present invention. System 10 incorporates a base or control unit 12 which can carry out many, if not all of the functions of a residential monitoring system.

Residential monitoring systems have been disclosed for example in U.S. Patent No. 6,402,691 B1 entitled In-Home Patient Monitoring System which issued June 11, 2002. Another such system has been disclosed in pending U.S. Patent Application No. 10/956,681 filed October 1, 2004 and entitled Mobile Telephonic Device and Base Station. The'681 application is assigned to the Assignee hereof and incorporated herein by reference.

Unit 12 includes a programmable processor 12a as well as interface circuitry 12b, which would be understood by those of skill in the art, for receiving information from members of a plurality 14 of physiological sensors. The members of the plurality 14 could include for example and without limitation physical condition sensors or monitors such as blood pressure monitors, heart rate monitors, temperature monitors, respiration sensors, activity monitors, sleep monitors, exercise monitors and the like all without limitation. Those of skill in the art will recognize that embodiments of the invention can include sensors or monitors of parameters of interest to healthy individuals such as runners, cyclists, weight lifters and the like, as well as those living with a chronic condition.

The sensors 14 communicate with the unit 12 by a wired or wireless medium indicated generally at 16. It will be understood that the nature and characteristics of the media of 16 are not limitations of the present invention.

Unit 12 incorporates control software 12c having a variety of different functional characteristics including data acquisition software 12c-1, communication software 12c-2, data and trend analysis software 12c-3, local display and interaction control software 12c-4. In accordance with the above description and as described in more detail subsequently, signals received from members of the plurality of sensors 14 can be acquired and analyzed by software 12c for purposes of informing residents, substantially in real-time, if desired, as to ongoing trends. Trend information can be accumulated in a data base 12d.

Trend information can be presented on a display unit 20. A resident who might be viewing trend information on the display 20 can interact with unit 12 via a touch screen or alternately mouse and/or keyboard 22. It will be understood that the exact characteristics of the display unit 20 or devices 22 are not limitations of the present invention.

Information relative to developing trends can be forwarded via a medium 26 to remote monitoring sites, friends or relatives. Medium 26 can include the switched telephone network, wired or wireless as well as computer networks such as the Internet.

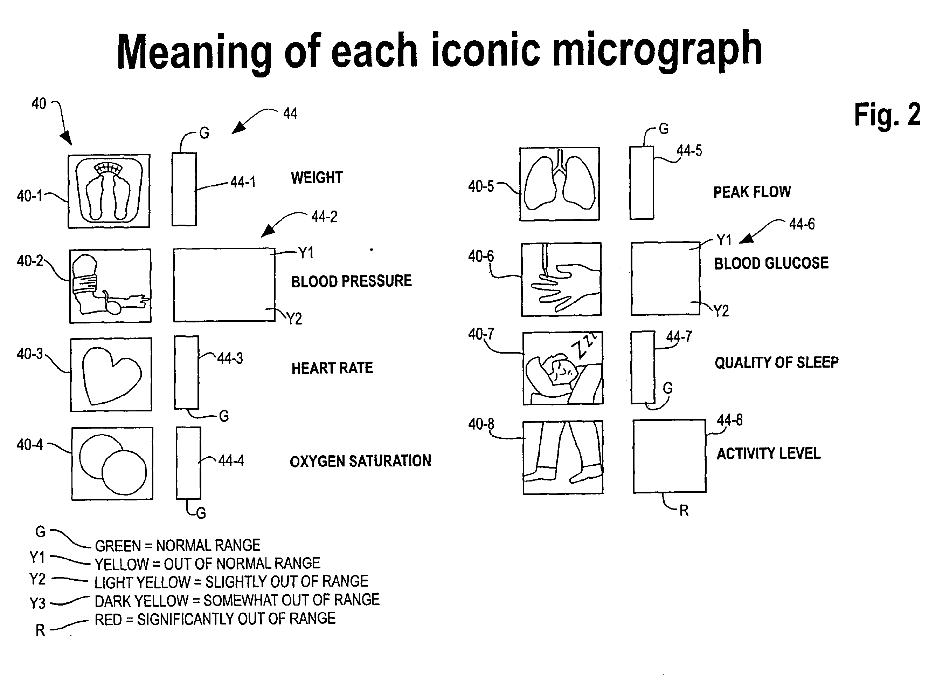

Fig. 2 illustrates various representative icons in accordance with the invention. A variety of such icons representative of differing physiological conditions can be presented, as discussed below in more detail, on the display 20 for viewing by the resident. Additionally, such information can be transmitted via the medium 26 to monitoring sites, relatives or friends as appropriate. It will be understood that the icons illustrated on Fig. 2 are exemplary only. Others come within the scope of the present invention.

Fig. 2 presents a plurality of icons 40 indicative of a variety of physiological conditions including weight, blood pressure, heart rate, oxygen saturation, peak blood flow, blood glucose level, quality of sleep and activity level. Each of the icons is representative of the associated physiological related parameter and can be presented without any need for an associated text label or description. The members of the plurality 40 can be presented in color or black and white.

It will be understood that a graphical user's interface could be provided so that a third party (a medical professional, coach or trainer) could specify the parameter(s) of interest and establish normal ranges associated therewith. All such specifications could be established remotely and downloaded via medium 26. Alternately, they could be established and entered locally into database 12d.

A plurality of condition indicating icons of 44 is associated with the members or the plurality 40. For example, icons 40-1, 40-3, 40-4, 40-5 and 40-7 have associated therewith respective state or condition indicating icons 44-1,44-3,44-4, 44-5 and 44-7.

Each of the iconic representations 44-1, 44-3, 44-4, 44-5 and 44-7 is presented, preferably, in green as indicative that the associated physiological parameter falls within a normal range. Normal range can be a range which is specific to the resident as indicated by prestored resident data 12d.

Other conditions have associated therewith indicating icons which make clear that the associated physiological parameter has varied from an expected or normal range. For example, exemplary parameter 40-2 for blood pressure has associated therewith a multi-toned yellow icon 44-2. The yellow colors are indicative of the fact that blood pressure has been varying between slightly out of range and out of normal range. Similarly, blood glucose level, indicated by icon 40-6 has associated therewith a multi-toned yellow icon 44-6 indicative of the fact that the resident's glucose level has been varying between slightly out of range and out of range. Finally, the resident's activity level, indicated by icon 40-8, has associated therewith a condition or quality indicator 44-8 which could be presented in red, indicative of the resident exhibiting a significant lower activity level than would be expected.

The condition indicating icons as well as the state or quality indicating icons, as illustrated above, could be presented with a variety of different symbols and colors without departing from the spirit and scope of the present invention. However, all such variations provide immediate graphical, visual feedback to the resident without a need to consider text messages.

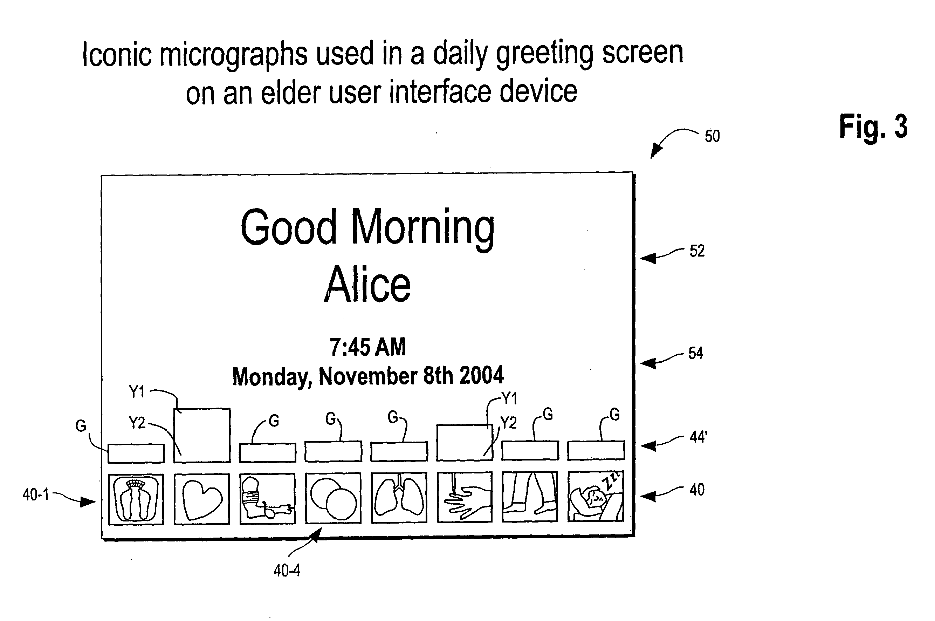

Fig. 3 illustrates one form of presentation of the family of icons of Fig. 2. Fig. 3 can be presented as a screen on the display 20.

As illustrated in a screen 50, Fig. 3, a customized introductory message 52 can be provided for the resident. The day and time 54 can also be provided. Finally, the plurality of icons 40 can be presented for the resident along with a plurality of condition indicating icons 44'. The resident can view at a glance the current state of a variety of physiological conditions without any need for reading textual messages or examining graphs.

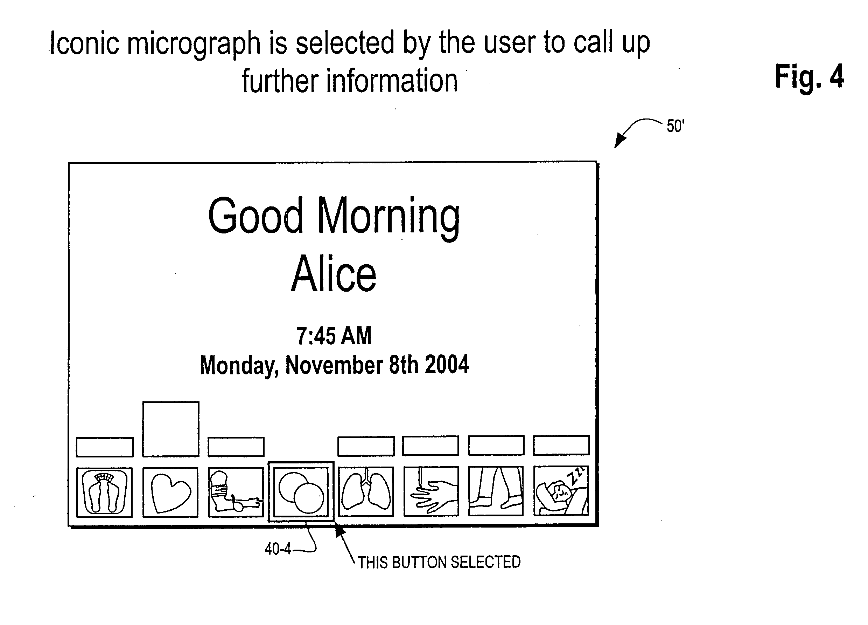

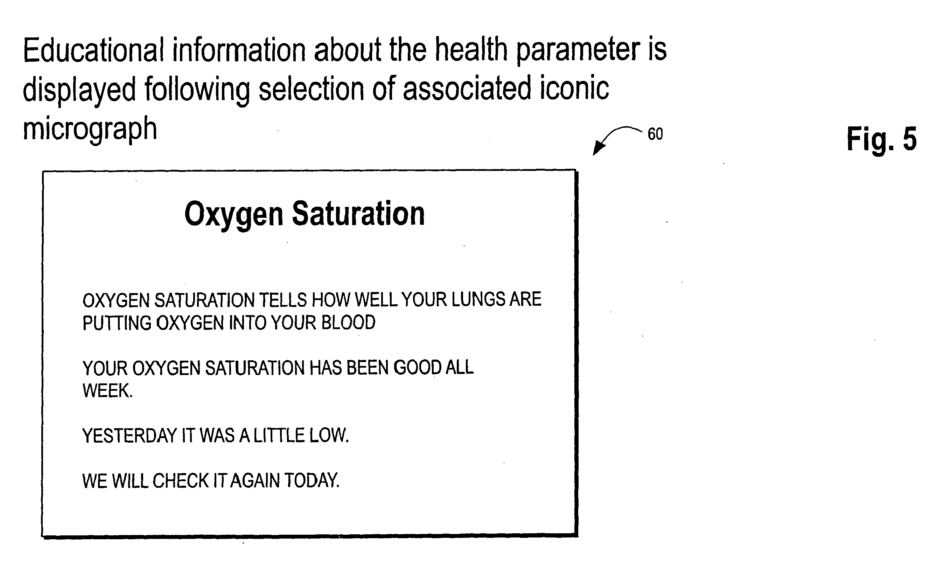

Where additional information is desired, as illustrated in Fig. 4, screen 50', a particular icon can be selected, such as the exemplary icon 40-4 which relates to an oxygen saturation level. The system 10 can respond as illustrated in Fig. 5 by presenting explanatory text, graphical information or educational video on a screen 60 for the resident. For example, explanations can be presented relative to parameters via selected output devices including speakers, monitors, or the like all without limitation.

From the foregoing, it will be observed that numerous variations and modifications may be effected without departing from the scope of the invention. It is to be understood that no limitation with respect to the specific apparatus illustrated herein is intended or should be inferred. It is, of course, intended to cover by the appended claims all such modifications as fall within the scope of the claims.

## Claims

1. A system comprising:
a display for visually presenting graphical images; and
a programmable processor coupled to the display, the processor executes
software that collects information as to the physical condition of an individual,
software that determines relationships between at least some of the collected information and members of a plurality of predetermined respective values, and
software, responsive to at least some of the determined relationships that presents on the display coded icons indicative of such relationships.

2. A system as in claim 1 where the executable software presents variable dimension, color coded icons on the display.

3. A system as in claim 2 which includes a plurality of physical parameter sensors.

4. A system as in claim 3 where the sensors are selected from a class that includes at least a temperature sensor, a respiratory sensor, a blood pressure sensor, a glucose sensor, an activity sensor, a blood oxygen level sensor and a heart rate sensor.

5. A system as in claim 3 which includes communications software that receives and responds to an inquiry from an individual relative to an icon presented on the display.

6. A system as in claim 4 which includes executable software that presents trend indicating audible feedback.

7. A system as in claim 4 which includes communications circuitry and software that transmits indicia pertaining to at least some of the icons to a displaced location.

8. A system as in claim 5 which includes executable software that receives information that specifies members of a plurality of conditions, and, at least one threshold value associated with each member of the plurality.

9. A system as in claim 8 which includes additional executable software that receives a second threshold value associated with at least one member of the plurality.
